# EUROPÄISCHE PATENTSCHRIFT

(11) **EP 3 319 701 B1**
(45) Veröffentlichungstag und Bekanntmachung des Hinweises auf die Patenterteilung: **15.04.2026**
(21) Anmeldenummer: 15777864.8
(22) Anmeldetag: 06.07.2015
(51) Int. Cl.: B01D 1/06, B01D 3/10, B01D 3/12, B01D 3/14, B01D 5/00, C11C 1/02, C11B 3/12, C07C 67/54

(54) **VERFAHREN ZUR GEWINNUNG VON FREIER UNGESÄTTIGTER FETTSÄURE UND/ODER FETTSÄUREESTER**
PROCESS FOR OBTAINING FREE UNSATURATED FATTY ACID AND/OR FATTY ACID ESTER
PROCÉDÉ D'OBTENTION D'ACIDES GRAS LIBRES INSATURÉS ET/OU D'ESTERS D'ACIDES GRAS

(43) Veröffentlichungstag der Anmeldung: 16.05.2018
(73) Patentinhaber: K.D. Pharma Bexbach GMBH, 66450 Bexbach (DE)
(72) Erfinder: KRUMBHOLZ, Rudolf, 66589 Merchweiler (DE)
(74) Vertreter: Patentanwälte Bernhardt / Wolff Partnerschaft mbB
(86) Internationale Anmeldenummer: PCT/DE2015/100280
(87) Internationale Veröffentlichungsnummer: WO 2017/005235

(56) Entgegenhaltungen:
- WO-A1-2012/048792
- US-A1- 2010 166 620
- US-A1- 2011 091 947
- "Verfahrenstechnik", 1969, OTTO KRAUSSKOPF VERLAG GMBH

## Beschreibung

Die Erfindung betrifft ein Verfahren zur Gewinnung von mehrfach ungesättigter freier Fettsäure und/oder Fettsäureester, bei dem zur Trennung eines Flüssigkeitsgemisches, das die mehrfach ungesättigte Fettsäure und/oder Fettsäureester enthält, ein aufsteigender Dampf des Flüssigkeitsgemischs in einer Kolonne einer Destillationsvorrichtung, die mindestens 10 theoretische Trennstufen aufweist, mit herabfließendem, aus dem Dampf gebildeten Kondensat in Kontakt gebracht wird, wobei auf Einbauten der Kolonne zwischen dem Dampf und dem Kondensat ein Stoff- und/oder Wärmeaustausch stattfindet.

US 2011/0091947 A1 beschreibt ein derartiges Verfahren für Omega-3-Fettsäuren, das folgenden Schritte umfasst: Herstellung eines Fettsäureethylesters (FAEE) durch Ethanolyse eines natürlichen Öls oder Fetts unter Verwendung von Ethanol in Gegenwart eines Enzymkatalysators; b) Unterziehen des hergestellten Fettsäureethylesters einer Vordestillation unter Verwendung einer Kurzweg-Destillationsvorrichtung; c) Bilden einer konzentrierten Fettsäure, indem man den Ethylester, der der Vordestillation unterzogen wurde, einer fraktionierten Destillation bei reduziertem Druck unterzieht; und d) Reinigen der konzentrierten Fettsäure mittels Simulated Moving Bed (SMB) Säulenchromatographie.

Aus der US 2010/0166620 A1 geht ein Verfahren zur kontinuierlichen Herstellung von Fettsäuremethylestern aus Fettsäuretriglyceriden aus Altöl unter Umesterung mittels eines wiederverwendbaren zuckerbasierten Katalysators hervor. Der Druckverlust zwischen Kopf und Sumpf einer Kolonne einer dabei verwendeten Destillationsvorrichtung beträgt 150 mbar.

Bei weiteren bekannten Verfahren wurde zur Durchführung wegen der großen Temperaturempfindlichkeit der freien Fettsäuren bzw. der Fettsäureester normalerweise eine, nur eine verhältnismäßig geringe Trennleistung erreichende, Kurzwegdestillation durchgeführt. Dabei wird aus einem dünnen Flüssigkeitsgemischfilm, der, ggf. durch Wischen, auf einer beheizten Verdampferfläche gebildet wird, auf einen nahe gegenüberliegenden Kondensator einer Kurzwegdestillationsvorrichtung partiell verdampft. Die geometrische Anordnung von der Verdampferfläche zur Kondensatorfläche sowie der kurze Abstand voneinander erlaubt Arbeitsdrücke im Druckbereich des Feinvakuums (1 mbar - 10⁻³ mbar) und somit entsprechend niedrige Verdampfungstemperaturen (vgl. Frank/Kutsche, "Die schonende Destillation" aus der Buchreihe "Verfahrenstechnik", Otto Krausskopf Verlag GmbH, Mainz, 1969).

Durch Benutzung ist ferner bekannt, zur Durchführung eines bekannten Verfahrens zur Gewinnung von freier Fettsäure und/oder von Fettsäureester eine Destillationseinrichtung zu verwenden, die einen Dünnschichtverdampfer und damit verbunden eine Rektifizierkolonne sowie einen Kondensator aufweist. Eine Verarbeitung der Fettsäure oder Fettsäureester enthaltenden Flüssigkeitsgemische wurde wegen der großen Temperaturempfindlichkeit dieser Stoffe bisher allerdings als problematisch angesehen.

Der Erfindung liegt die Aufgabe zugrunde, die Effizienz des eingangs genannten Verfahrens zu erhöhen.

Erfindungsgemäß wird diese Aufgabe dadurch gelöst, dass die Trennung mit einem Druckverlust zwischen Kopf und Sumpf der Kolonne Δp, der ≥ 3,3 mbar und ≤ 6 mbar ist, durchgeführt wird.

Überraschend hat sich gezeigt, dass sich bei Durchführung des erfindungsgemäßen Verfahrens trotz der für den verhältnismäßig großen Druckverlust notwendigen hohen Temperaturen in der Destillationsvorrichtung keine Zersetzung oder Isomerisierung der Fettsäure bzw. Fettsäureester stattfindet. Vorteilhaft kann mit dem Verfahren bei guter Trennwirkung im Vergleich zu den bekannten Verfahren ein größerer Durchsatz erreicht werden.

Das erfindungsgemäße Verfahren eignet sich insbesondere zur Verarbeitung von mehrfach ungesättigter Fettsäure wie Omega-6- oder Omega-3-Fettsäure, beispielsweise ALA (9Z,12Z,15Z)-Octadeca-9,12,15-triensäure, SDA (6Z,9Z, 12Z, 15Z)-Octadeca-6,9,12,15-tetraensäure, ETA (8Z,11Z,14Z,17Z)-Eicosa-8,11,14,17-tetraensäure, EPA (5Z,8Z,11Z,14Z,17Z)-Eicosa-5,8,11,14,17-pentaensäure, HPA (6Z,9Z,12Z,15Z,18Z)-Heneicosa-6,9,12,15,18-pentaensäure, DPA (7Z,10Z,13Z,16Z,19Z)-Docosa-7,10,13,16,19-pentaensäure, DHA (4Z,7Z,10Z,13Z,16Z,19Z)-Docosa-4,7,10,13,16,19-hexaensäure. Solche Flüssigkeitsgemische werden zweckmäßigerweise aus Pflanzenöl oder/und aus Fischöl und/oder aus Mikroorgansimen, z.B. Mikroalgen, Hefe oder Bakterien, hergestellt.

In einer bevorzugten Ausführungsform der Erfindung weist die Kolonne mindestens 30, vorzugsweise mindestens 40, theoretische Trennstufen auf.

In einer Ausgestaltung der Erfindung wird die Trennung mit einem Druckverlust Δp durchgeführt, der 3,5 mbar ≤ Δp beträgt.

Zweckmäßigerweise beträgt der F-Faktor der Kolonne dabei höchstens 2 Pa^{½}, vorzugsweise höchstens 1,5 Pa^{½} , besonders bevorzugt höchstens 1,1 Pa^{½}.

In einer Ausgestaltung der Erfindung umfasst die Destillationsvorrichtung, mit der das erfindungsgemäße Verfahren durchgeführt wird, einen Dünnschichtverdampfer. Die genannten Einbauten umfassen zweckmäßigerweise Böden, vorzugsweise Siebböden, Glockenböden oder Ventilböden, oder Füllkörper und/oder Packungen aus Blech oder aus Drahtgewebe auf. Ein Kondensator der Destillationsvorrichtung ist zweckmäßigerweise durch einen Totalkondensator gebildet.

In einer Weiterbildung der Erfindung wird ein durch die Trennung mit dem beschriebenen Verfahren im Sumpf der Destillationsvorrichtung gewonnenes Sumpfprodukt einer Harnstoff-Fällung unterzogen. Bei dieser aus dem Stand der Technik bekannten Harnstoff-Fällung wird ein Gemisch von Ethanol und Harnstoff zum Sieden gebracht und das Sumpfprodukt unter Rühren hinzugegeben. Nachdem abkühlen gelassen worden ist wird ein dabei ausgefallener HarnstoffKuchen durch Filtration abgetrennt. Aus dem daraus verbleibenden Gemisch wird enthaltendes Ethanol abdestilliert und kann ggf. für weitere Fällungen verwendet werden. Das danach verbleibende Gemisch wird einer, ebenfalls aus dem Stand der Technik bekannte, Kurzwegdestillation unterzogen.

In einer Ausgestaltung der Erfindung wird ein durch die Trennung im Kopf der Destillationsvorrichtung gewonnenes Kopfprodukt erneut mit dem oben beschriebenen Verfahren verarbeitet und ein dabei gewonnenes Zweitsumpfprodukt mit einem mittels der Kurzwegdestillation gewonnenem Zweitkopfprodukt gemischt.

Vorteilhaft lässt sich das Flüssigkeitsgemisch mittels der Harnstoff-Fällung, der Kurzwegdestillation sowie der erneuten Trennung des Kopfprodukts mit der freien Fettsäure bzw. dem Fettsäureester stark anreichern. Beispielsweise lassen sich mit EPA oder DPA hoch angereicherte Omega-3-Fettsäuren herstellen.

Die Erfindung wird nachfolgend anhand von Ausführungsbeispielen und der beigefügten Zeichnung näher erläutert.

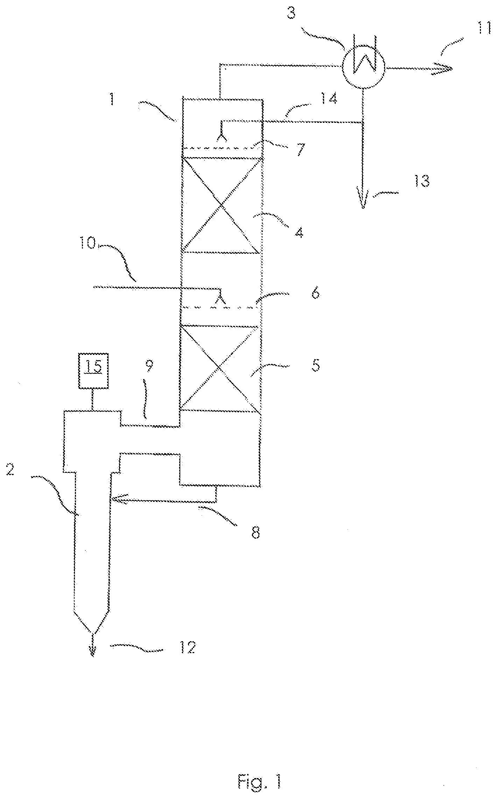

Zur Durchführung einer Rektifikation wurde eine in Fig. 1 schematisch dargestellte Destillationsvorrichtung verwendet, die einen mit einem Motor 15 versehenen Dünnschichtverdampfer 2, einen mit dem Dünnschichtverdampfer 2 verbundenen und mit Drahtgewebepackungen 4,5 belegte Rektifizierkolonne 1 sowie ein Kondensator 3 umfasste. Die Rektifizierkolonne 1 wies einen F-Faktor von 1 Pa^{½} auf.

Zwischen einem Boden der Rektifizierkolonne 1 und dem Dünnschichtverdampfer 2 ist eine Leitung 8 vorgesehen, über die der Sumpf dem Dünnschichtverdampfer 2 zugeführt wird. Im Dünnschichtverdampfer 2 erzeugte Dämpfe gelangen über ein Brüdenrohr 9, das den Dünnschichtverdampfer 2 mit der Rektifizierkolonne 1 verbindet, in die Rektifizierkolonne 1, wobei die Dampftemperatur in über dem Kolonnensumpf 180 bis 210 °C beträgt. Ein nicht verdampfter Anteil wird als Sumpfprodukt 12 vom Dünnschichtverdampfer 2 abgeführt. Im unteren Teil der Rektifizierkolonne l ist die Drahtgewebepackung 5 (Fa. Montz, Hilden) angeordnet, die als Abtriebsteil wirkt. Im oberen Teil der Kolonne befindet sich die Drahtgewebepackung 4 (Fa. Montz, Hilden), die als Verstärkungssteil wirkt. Über eine Zuführungsleitung 10, die zwischen den Drahtgewebepackungen 4,5 in die Rektifizierkolonne 1 mündet, wird ein Aufgabematerial eingespeist und mit Hilfe einer Verteileinrichtung 6 auf der gleichmäßig auf der Drahtgewebepackung 5 verteilt. Am Kopf der Rektifizierkolonne 1 befindet sich ein Kondensator 3, mittels dessen die am Kopf der Rektifizierkolonne 1 austretenden Dämpfe kondensiert werden. Ein Teil des entstandenen Kondensats wird über eine Rückflussleitung 14 in die Kolonne zurückgeführt und mit Hilfe einer weiteren Verteileinrichtung 7 gleichmäßig auf der Drahtgewebepackung 4 verteilt. Nicht zurückgeführtes Kondensat wird als Destillat wird über eine Abführleitung 13 aus der Destillationsvorrichtung gefördert. Eine Vakuumleitung 11 führt zu einem mehrstufigen Vakuumpumpstand, der Drücke im Kopf der Fraktionierkolonne 1 von bis zu ca. 0,1 mbar erzeugen kann.

Die Destilliervorrichtung kann mit Durchsätzen von ca. 100 bis 600 kg/h arbeiten. Für die nachfolgend wiedergegebenen Versuche wurde mit einem Durchsatz von 400 kg/h gearbeitet.

In den nachfolgend wiedergegebenen Ergebnissen steht EE für Ethylester,

### Beispiel 1:

Ein zu verarbeitendes Flüssigkeitsgemisch war ein Aufgabeöl, das vor der Destillation 310 mg/g EPA-EE und 200 mg/g DHA-EE enthielt.

Nach Destillation mit der beschriebenen Destillationsvorrichtung bei einer Druckdifferenz zwischen Sumpf und Kopf der Destillationsvorrichtung von Δp = 3,9 mbar wurden bei einer Anzahl von ca. 50 theoretischen Böden und bei 400 kg/h Aufgabe 73 % Sumpfprodukt und 27 % Kopfprodukt gewonnen, wobei das Sumpfprodukt 370 mg/g EPA-EE und 270 mg/g DHA- EE und das Kopfprodukt 150 mg EPA-EE und 1,5 mg/g DHA-EE enthielt.

### Beispiel 2:

Ein zu verarbeitendes Flüssigkeitsgemisch war ein Aufgabeöl, das vor der Destillation 360 mg/g EPA-EE und 235 mg/g DHA-EE enthielt. Nach Destillation mit der beschriebenen Destillationsvorrichtung bei einer Druckdifferenz zwischen Sumpf und Kopf der Destillationsvorrichtung Δp = 3,4 mbar wurden bei einer Anzahl von ca. 50 theoretischen Böden und bei 400 kg/h Aufgabe 76 % Sumpfprodukt und 24 % Kopfprodukt gewonnen. Das Sumpfprodukt enthielt 390 mg/g EPA-EE und 310 mg/g DHA-EE, das HPE- Kopfprodukt 255 mg EPA-EE und 1 mg/g DHA-EE.

### Beispiel 3:

Ein zu verarbeitendes Flüssigkeitsgemisch war ein Aufgabeöl, das vor der Destillation 420 mg/g EPA-EE und 210 mg/g DHA-EE enthielt. Nach Destillation mit der beschriebenen Destillationsvorrichtung bei einer Druckdifferenz zwischen Sumpf und Kopf der Destillationsvorrichtung Δp = 3.6 mbar wurden bei einer Anzahl von ca. 50 theoretischen Böden und bei 400 kg/h Aufgabe 74 % Sumpfprodukt und 26 % Kopfprodukt gewonnen. Das Sumpfprodukt enthielt 430 mg/g EPA-EE und 280 mg/g DHA-EE, das Kopfprodukt 400 mg EPA-EE und 15 mg/g DHA-EE.

### Beispiel 4:

Ein zu verarbeitendes Flüssigkeitsgemisch war ein Aufgabeöl, das vor der Destillation 430 mg/g EPA-EE und 145 mg/g DHA-EE enthielt. Nach Destillation mit der beschriebenen Destillationsvorrichtung bei einer Druckdifferenz zwischen Sumpf und Kopf der Destillationsvorrichtung Δp = 4,1 mbar wurden bei einer Anzahl von ca. 50 theoretischen Böden und bei 400 kg/h Aufgabe 70 % Sumpfprodukt und 30 % Kopfprodukt gewonnen. Das Sumpfprodukt enthielt 510 mg/g EPA-EE und 205 mg/g DHA-EE, das Kopfprodukt 230 mg/g EPA-EE und 2 mg/g DHA-EE.

### Beispiel 5:

Ein zu verarbeitendes Flüssigkeitsgemisch war ein Aufgabeöl, das vor der Destillation 310 mg/g EPA-EE und 200 mg/g DHA-EE enthielt.

Nach Destillation mit der beschriebenen Destillationsvorrichtung bei einer Druckdifferenz zwischen Sumpf und Kopf der Destillationsvorrichtung Δp = 3,9 mbar wurden bei einer Anzahl von 50 theoretischen Böden und bei 400 kg/h Aufgabe 73 % Erstsumpfprodukt und 27 % Erstkopfprodukt gewonnen, wobei das Erstsumpfprodukt 370 mg/g EPA-EE und 270 mg/g DHA- EE und das Erstkopfprodukt 150 mg EPA-EE und 1,5 mg/g DHA-EE enthielt.

Das Erstsumpfprodukt wurde nachfolgend einer Harnstofffällung unterzogen. Dabei wurde zunächst ein Gemisch von drei Teilen Ethanol und einem Teil Harnstoff zum Sieden gebracht und dann das Erstsumpfprodukt unter Rühren dazugegeben. Nach Abkühlen dieser Mischung wurde ein aus der Mischung ausgefallener Harnstoffkuchen durch Filtration abgetrennt und aus einem verbleibenden Gemisch das Ethanol abdestilliert. Das danach verbleibende Gemisch wurde anschließend einer Kurzwegdestillation bei einer Einspeisemenge von 200 kg/h, einer Verdampfermanteltemperatur von 180 bis 205 Grad Celcius bei einem Druck von 0,01 bis 0,1 mbar unterzogen. Das mittels der Kurzwegdestillation erhaltene Zweitkopfprodukt enthielt 490 mg/g EPA-EE und 310 mg/g DHA-EE.

Das Erstkopfprodukt wurde erneut mit einer erfindungsmäßen Destillationsvorrichtung destilliert. Das dadurch gewonnene Zweitsumpfprodukt enthielt eine Fettsäureethylester-Mischung mit 610 mg/g EPA-EE und 6 mg/g DHA-EE.

Durch Mischen des Zweitsumpfprodukts und des Zweitkopfprodukts im Verhältnis 40:60 wurde anschließend als Zwischenprodukt eine Fettsäureethylester-Mischung mit 538 mg/g EPA-EE und 188 mg/g DHA-EE erhalten. Diese wurde verseift, indem sie mit Ethanol (96%ig) sowie 0,617 kg NaOH vermischt und auf 60°C erwärmt wurde. Die Mischung wird nach einer Stunde auf Raumtemperatur abgekühlt und mit verdünnter Schwefelsäure auf einen pH Wert von 5 eingestellt. Die wässrige Phase und die Öl-Phase wurden voneinander getrennt und die Öl-Phase gewaschen. Anschließend wurde die Öl-Phase unter Vakuum destilliert, so dass der Wassergehalt in der Öl-Phase unter 0,1% beträgt.

Damit ließ sich mittels anschließender Ansäuerung ein Produkt mit der Zusammensetzung EPA 538 mg/g und DHA 188 mg/g in der Form der freien Fettsäure gewinnen.

### Beispiel 6:

Ein zu verarbeitendes Flüssigkeitsgemisch war ein Aufgabeöl, das vor der Destillation 320 mg/g EPA-EE und 200 mg/g DHA-EE enthielt. Nach Destillation mit der beschriebenen Destillationsvorrichtung bei einer Druckdifferenz zwischen Sumpf und Kopf der Destillationsvorrichtung Δp = 3,5 mbar wurden bei einer Anzahl von ca. 50 theoretischen Böden und bei 400 kg/h Aufgabe 74 % Sumpfprodukt und 26 % Kopfprodukt gewonnen. Das Sumpfprodukt enthielt 370 mg/g EPA-EE und 270 mg/g DHA-EE, das HPE- Kopfprodukt 190 mg EPA-EE und 1 mg/g DHA-EE.

Das Erstsumpfprodukt wurde nachfolgend wie oben beschrieben einer Harnstoff-fällung und anschließend einer Kurzwegdestillation unterzogen. Das so mittels der Kurzwegdestillation erhaltene Zweitkopfprodukt enthielt 490 mg/g EPA-EE und 310 mg/g DHA-EE.

Das Erstkopfprodukt wurde erneut mit einer erfindungsmäßen Destillationsapparatur destilliert. Das dadurch gewonnene Zweitsumpfprodukt enthielt eine Fettsäureethylester-Mischung mit 630 mg/g EPA-EE und 3 mg/g DHA-EE.

Durch Mischen des Zweitsumpfprodukts und des Zweitkopfprodukts im Verhältnis 40:60 wurde anschließend als Endprodukt eine Fettsäureethylester-Mischung mit 546 mg/g EPA-EE und 187 mg/g DHA-EE erhalten.

## Patentansprüche

1. Verfahren zur Gewinnung von mehrfach ungesättigter freier Fettsäure und/oder Fettsäureester und, bei dem zur Trennung eines Flüssigkeitsgemisches, das die mehrfach ungesättigte Fettsäure und/oder den Fettsäureester enthält, ein aufsteigender Dampf des Flüssigkeitsgemischs in einer Kolonne einer Destillationsvorrichtung, die mindestens 10 theoretische Trennstufen aufweist, mit herabfließendem, aus dem Dampf gebildeten Kondensat in Kontakt gebracht wird, wobei auf Einbauten der Kolonne zwischen dem Dampf und dem Kondensat ein Stoff- und Wärmeaustausch stattfindet,
**dadurch gekennzeichnet,**
**dass** die Trennung mit einem Druckverlust zwischen Kopf und Sumpf der Kolonne Δp, der ≥ 3,3 mbar und ≤ 6 mbar ist, durchgeführt wird.

2. Verfahren nach Anspruch 1,
**dadurch gekennzeichnet,**
**dass** das Flüssigkeitsgemisch Omega-6- oder Omega-3-Fettsäure und/oder Alkyl- und/oder Glycerinmonoester enthält.

3. Verfahren nach Anspruch 1 oder 2,
**dadurch gekennzeichnet,**
**dass** das Flüssigkeitsgemisch aus Pflanzenöl oder/und aus Fischöl und/oder aus Mikroorgansimen hergestellt wird.

4. Verfahren nach Anspruch 1 oder 3,
**dadurch gekennzeichnet,**
**dass** die Kolonne mindestens 30, vorzugsweise mindestens 40, theoretische Trennstufen aufweist.

5. Verfahren nach einem der Ansprüche 1 bis 4,
**dadurch gekennzeichnet,**
**dass** die Trennung mit einem Druckverlust Δp ≥ 3,5 mbar durchgeführt wird.

6. Verfahren nach einem der Ansprüche 1 bis 5,
**dadurch gekennzeichnet,**
**dass** der F-Faktor die Kolonne höchstens 2 Pa^{½} , vorzugsweise höchstens 1,5 Pa^{½}, besonders bevorzugt höchstens 1,1 Pa^{½} , beträgt.

7. Verfahren nach einem der Ansprüche 1 bis 6,
**dadurch gekennzeichnet,**
**dass** das Flüssigkeitsgemisch mit einem Dünnschichtverdampfer der Destillationsvorrichtung verdampft wird.

8. Verfahren nach einem der Ansprüche 1 bis 7,
**dadurch gekennzeichnet,**
**dass** ein durch die Trennung im Sumpf der Destillationsvorrichtung gewonnenes Sumpfprodukt einer Harnstoff-Fällung und anschließend einer Kurzwegdestillation unterzogen wird.

9. Verfahren nach Anspruch 8,
**dadurch gekennzeichnet,**
**dass** ein durch die Trennung im Kopf der Destillationsvorrichtung gewonnenes Kopfprodukt erneut mit dem Verfahren nach einem der Ansprüche 1 bis 8 verarbeitet wird und ein dabei gewonnener Zweitsumpfprodukt mit einem mittels der Kurzwegdestillation gewonnenem Zweitkopfprodukt gemischt wird.

## Claims

1. Process for obtaining polyunsaturated free fatty acid and/or fatty acid ester, wherein in order to separate a liquid mixture containing the polyunsaturated fatty acid and/or the fatty acid ester, ascending vapor of the liquid mixture is brought into contact in a column of a distillation apparatus having at least 10 theoretical plates with downflowing condensate formed from the vapor, with mass transfer and heat transfer taking place between the vapor and the condensate on internals of the column,
**characterized**
**in that** the separation is carried out at a pressure drop between top and bottom of the column Δp which is ≥ 3.3 mbar and ≤ 6 mbar.

2. Process according to Claim 1,
**characterized**
**in that** the liquid mixture contains omega-6 or omega-3 fatty acid and/or alkyl and/or glyceryl monoesters.

3. Process according to Claim 1 or 2,
**characterized**
**in that** the liquid mixture is produced from vegetable oil and/or from fish oil and/or from microorganisms.

4. Process according to Claim 1 or 3,
**characterized**
**in that** the column has at least 30, preferably at least 40, theoretical plates.

5. Process according to any one of Claims 1 to 4,
**characterized**
**in that** the separation is carried out at a pressure drop Δp of ≥ 3.5 mbar.

6. Process according to any one of Claims 1 to 5,
**characterized**
**in that** the F factor of the column is not more than 2 Pa^{½}, preferably not more than 1.5 Pa^{½}, particularly preferably not more than 1.1 Pa^{½}.

7. Process according to any one of Claims 1 to 6,
**characterized**
**in that** the liquid mixture is vaporized by means of a thin film evaporator of the distillation apparatus.

8. Process according to any one of Claims 1 to 7,
**characterized**
**in that** a bottom product obtained at the bottom of the distillation apparatus as a result of the separation is subjected to a urea precipitation and subsequently a short path distillation.

9. Process according to Claim 8,
**characterized**
**in that** an overhead product obtained at the top of the distillation apparatus as a result of the separation is again processed using the process according to any of Claims 1 to 8 and a second bottom product obtained in this way is mixed with a second overhead product obtained by means of the short path distillation.

## Revendications

1. Procédé d'obtention d'acides gras libres polyinsaturés et/ou d'esters d'acides gras, dans lequel, pour séparer un mélange liquide contenant les acides gras polyinsaturés et/ou les esters d'acides gras, une vapeur ascendante du mélange liquide est mise en contact avec un condensat descendant formé à partir de la vapeur, dans une colonne d'un dispositif de distillation comportant au moins 10 plateaux de séparation théoriques, un échange de matière et de chaleur ayant lieu entre la vapeur et le condensat sur des internes de la colonne,
**caractérisé en ce que** la séparation est effectuée avec une perte de charge entre la tête et le bas de la colonne Δp, qui est ≥ 3,3 mbar et ≤ 6 mbar.

2. Procédé selon la revendication 1,
**caractérisé en ce que** le mélange liquide contient des acides gras oméga-6 ou oméga-3 et/ou des mono-esters d'alkyle et/ou de glycérol.

3. Procédé selon la revendication 1 ou 2,
**caractérisé en ce que** le mélange liquide est produit à partir d'huile végétale et/ou d'huile de poisson et/ou de micro-organismes.

4. Procédé selon la revendication 1 ou 3,
**caractérisé en ce que** la colonne comporte au moins 30, de préférence au moins 40, plateaux de séparation théoriques.

5. Procédé selon l'une des revendications 1 à 4,
**caractérisé en ce que**
la séparation est effectuée avec une perte de charge Δp ≥ 3,5 mbar.

6. Procédé selon l'une des revendications 1 à 5,
**caractérisé en ce que** le facteur F de la colonne est au maximum de 2 Pa^{½}, de préférence au maximum de 1,5 Pa^{½}, et de manière particulièrement préférée au maximum de 1,1 Pa^{½}.

7. Procédé selon l'une des revendications 1 à 6,
**caractérisé en ce que** le mélange liquide est évaporé à l'aide d'un évaporateur à couche mince du dispositif de distillation.

8. Procédé selon l'une des revendications 1 à 7,
**caractérisé en ce qu'**un produit de bas de colonne obtenu par la séparation en bas du dispositif de distillation est soumis à une précipitation d'urée, puis à une distillation à court trajet.

9. Procédé selon la revendication 8,
**caractérisé en ce qu'**un produit de tête de colonne obtenu par la séparation en tête du dispositif de distillation est à nouveau traité par le procédé selon l'une des revendications 1 à 8, et un deuxième produit de bas de colonne ainsi obtenu est mélangé à un deuxième produit de tête de colonne obtenu par distillation à court trajet.
